# EUROPEAN PATENT APPLICATION

(11) **EP 0 796 621 A2**
(43) Date of publication of application: **24.09.1997**
(21) Application number: 97301866.6
(22) Date of filing: 19.03.1997
(51) Int. Cl.: A61K 35/78

(54) **Preventing and treating periodontal disease**

(30) Priority: 19.03.1996 JP 62506/96
(71) Applicant: Matsuda, Shimpei, Naka-gun, Ibaraki-ken (JP)
(72) Inventor: Matsuda, Shimpei, Naka-gun, Ibaraki-ken (JP)
(74) Representative: Sheard, Andrew Gregory

(57) **Abstract**

A tobacco extract, or one or more chemical compounds characteristic of a tobacco extract, is useful in the prevention or treatment of periodontal disease. The tobacco extract may be obtained from tobacco leaf, part-processed tobacco leaf or tobacco for smoking, in an aqueous solution, for example by gas or liquid phase extraction. Alternatively the compounds may be chemically synthesized. The medicinally active agent may be a chemical compound containing nitrogen, and it may be one or a mixture of alkaloids, terpenoids and carotenoids. The use of a tobacco extract or one or more chemical compounds characteristic of tobacco extract in the preparation of a medicament for the prevention or treatment of periodontal disease is also claimed. The extract may be in the form of solution, paste, jelly, gum or solid.

## Description

This invention relates to prevention and treatment of periodontal disease. More particularly, the invention relates to the prevention and treatment of periodontal disease using tobacco extract.

Among the many diseases which humans suffer from, there are diseases relating to teeth and gums such as teeth decay and periodontal disease. Periodontal disease causes bad breath and results in the loss of teeth at an advanced stage. Thus, periodontal disease is unpleasant for sufferers as well as for the people around them. A method or a medicine to cure periodontal disease has never been known nor practised.

It is an object of the present invention to provide a composition for the prevention and treatment of periodontal disease.

It is a further object of the present invention to provide a composition to be effective against a variety of bacteria causing periodontal disease.

Further objects of the present invention will be understood from the following description.

According to a first aspect of the invention, there is provided a tobacco extract for preventing or treating periodontal disease.

According to a second aspect of the invention, there is provided one or more chemical compounds characteristic of a tobacco extract for preventing or treating periodontal disease.

According to a third aspect of the invention, there is provided a method of producing a tobacco extract as described above, the method comprising preparing the extract by gas or liquid phase extraction.

According to a fourth aspect of the invention, the use of a tobacco extract in the preparation of a medicament for the prevention or treatment of periodontal disease is provided.

According to a fifth aspect of the invention, the use of one or more chemical compounds characteristic of a tobacco extract in the preparation of a medicament for the prevention or treatment of periodontal disease is provided.

Preferred embodiments of the invention will now be described.

To explain the present invention clearly, the construction of a tooth will be briefly described. The supporting structure of teeth is called the periodontal tissue, and it is essentially composed of:
Root (or gum)
Periodontal Membrane
Cement
Alveolar Bone

Diseases of the periodontal tissue are collectively called periodontal disease. When the disease is limited to the gum, it is called gingivitis, and when the disease reaches to the alveolar bone, it is called periodonitis. In this specification, these diseases are referred to collectively as periodontal disease. The present invention relates to the prevention and treatment of periodontal disease.

There exists a thin gap called the gingival gap between the teeth and gums, ranging from 0.1 to 1.5 mm in thickness even for healthy teeth. A variety of bacilli breed in the gingival gap, because nutrition is continuously being supplied. Unless the mouth is kept extraordinarily clean, residues of food and bacilli will give rise to dental plaque (bacterial plaque). The main constituent bacilli in the dental plaque known are *Streptococcus, Actinomyces, Nocardia, Veillonella, Neisseria, Bacteroides, Fusobacterium,* and *Leptotrichia.* The dental plaque is a composite of food residues and bacilli whose metabolic products are the main cause of the periodontal disease. Many kinds of bacilli are known to live in the gingival gap, excreting through the metabolic process various undesirable chemicals which cause inflammation of the gum. In the gingivitis state reddening and inflammation of the gum tissue is commonly observed. When the inflammation spreads to deeper structures of teeth and eventually damages the alveolar bone, it is called periodontitis. In the periodontitis state, a periodontal pocket is formed which discharges pus in addition to the inflammation. In recent years, bacilli like *Bacteroides, Fusobacterium, Capnocytophaga, Actinobacillus* and *Eikenella* are noted as causing periodontal disease.

As explained above, periodontal disease is caused by bacilli breeding in the gingival gap. In order to deal with the root of periodontal disease, we have invented a method which sterilises or stops the breeding of bacilli. Although it would obviously be better to keep the mouth very clean and not to supply any nutrition to the bacilli, this will require prolonged brushing of teeth by a toothbrush. For the ordinary man to prevent and treat periodontal disease, therefore, invention of medicinal materials to sterilise or suppress the breeding of periodontal bacilli is awaited. At the present time (April 1996), there is a commercially available dental paste (for example, trade name GUM which contains cetylpyridinium chloride as a medicinal component) which claims to fight periodontal bacilli. The present inventor has used it for several years, but it has failed to cure an existing periodontal disease. It is said that periodontal disease can be effectively prevented whereas to cure it is more difficult. It is generally admitted that there is no dental paste currently available on the market for the cure of periodontal disease. An objective of the present invention is to provide a material which can prevent or cure periodontal disease.

The present inventor is a male Japanese of 53 years (as of December 1995) and has suffered from periodontal disease since around 35 years old, resulting in the loss of several teeth. Since then the inventor has had bad breath. He tried to keep his mouth clean by brushing after each meal, had the dental calculus removed by a dentist regularly and used dental floss in addition to a toothbrush, but he failed to cure the periodontal disease.

The present inventor has been smoking since he was 18 years old. He has also quitted smoking many times, about 20 times for the duration of 1 to 5 weeks, and 3 times for the duration of 1 to 3 years. As stated above, he has suffered from periodontal disease since around 35 years old and he has noticed a correlation between the severity of the periodontal disease and the quitting of smoking. Gum inflammation, unpleasant odour and loosening of teeth were experienced generally after quitting smoking for one to two months. Discharge of pus, which is a symptom of periodontitis, was observed in worse cases. When smoking was resumed, the gum inflammation as well as the unpleasant odour subsided gradually in a few weeks. When the non-smoking phase lasted for longer than one year, the inflammation became worse and periodontitis occurred, resulting in the loss of two teeth. From these experiences, it was deduced that tobacco or a substance from tobacco is effective for preventing periodontal disease. The present inventor applied an extract from tobacco to his teeth with a toothbrush and discovered that it prevented and cured the periodontal disease, which led to the present invention. The present invention provides a tobacco extract for the prevention and treatment of periodontal disease.

An object of the present invention is to provide a material and a method of preparing it for the prevention or treatment of periodontal disease. The medicinally active agents are contained in tobaccos (cured tobaccos or cigarettes) and tobacco leaf. As described in detail later, tobacco (for smoking), tobacco leaf and smoke from tobacco contain many kinds of chemicals and substances. The medicinally effective agent in the present invention is one or more compounds extracted from tobacco. Since there are many kinds of bacilli that cause periodontal disease, it is conceivable that one compound or the concerted effect of many compounds works against periodontal disease. A particular compound from tobacco extract may sterilize a bacillus, while not affecting others. In these cases, it is desirable to use several different kinds of extracts from various tobaccos alternately.

The best known components of tobacco smoke are nicotine and tar. The tobacco extracts of the present invention are not limited to these two components. Commercially available tobacco for smoking is manufactured mainly from *Nicotiana tabacum* of *Solanum melongena* and minorly from *Nicotiana latissima* and *Nicotiana rustica.* In the present invention, these are collectively called "tobacco". The starting material of the tobacco extract of the present invention includes commercial tobaccos such as cigarettes, cigars, chewing tobaccos, leaf tobaccos, part-processed tobacco leaf, and tobaccos produced by cell culture. In the literature, tobacco is classified into (1) tobacco or cigarettes for smoking, (2) leaf tobacco or a raw material for cigarettes, or (3) tobacco plants or tobacco leaf as the botanical species. In the present invention, the term "tobacco" is used to represent all of these.

Among the many constituents of tobacco, the most characteristic are the alkaloids, i.e. botanical alkaline compounds containing nitrogen. Twelve alkaloids are known to exist in tobacco, among which nicotine, nornicotine, and anabacine are most familiar. It is generally known that alkaloids have medicinal or pharmaceutical effects and sometimes toxic effects. Although 65 kinds of nicotiana plants are known, only *Nicotiana tabacum, Nicotiana latissima* and *Nicotiana rustica* are currently cultivated. Major types of *Nicotiana tabacum* grown commercially are the flue-cured tobacco, burley tobacco, orient tobacco, and domestic (in Japan) tobacco. These species are all used as raw materials of the tobacco extract in the present invention. After harvesting, tobacco leaves are dried by flue-curing or air curing. The water content of the dried tobacco leaves are adjusted and homogenized, and then the leaves are put through an ageing process. In the present invention, tobacco leaf and part-processed tobacco leaf can be used as raw material for the extract. In addition, tobacco as a plant can also be used as the raw material.

In the present invention, an extract of tobacco is contacted with the teeth and gum to prevent and treat periodontal disease. Although it has not been identified which chemical compounds in the extract have the medicinal effect, it is conceivable that one or more chemical substances in the extract are responsible. It is also conceivable that there is a compounded or concerted effect arising from a mixture of several chemical compounds in the extract.

Several scientific papers describe some of the chemicals in tobacco leaf and tobacco smoke, and have shown the existence of several thousands or more. Tobacco smoke is considered to be the product of heated gas phase extraction of tobacco. An example of the composition of tobacco smoke is given below:

| | |
|---|---|
| Nitrogen | 59% |
| Oxygen | 13% |
| Carbon dioxide | 13% |
| Carbon monoxide | 3% |
| Total particulate matters | 8% |
| Vapour phase | 3% |
| Others | Remainder |

The composition of the particulate matters is given below:

| | |
|---|---|
| Carboxilic acid | 13% |
| Aldehydes and ketones | 11% |
| Alcohols | 8% |
| Nicotine | 6% |
| Alkaloids (other than nicotine) | 3.5% |
| Esters | 3.5% |
| Phenols | 3.5% |
| Terpenes | 5% |
| Alkane | 5% |
| Others | Remainder |

As stated above tobacco smoke is considered to be the product of thermal decomposition of tobacco. The chemical composition of smoke has been studied in relation to its taste and flavour, and found to be further complicated and diversified. The chemical composition of volatile constituents from the particulate matters is exemplified below:
(1) Hydrocarbons
   Alkane such as methane and aromatic hydrocarbons such as benzene, isoprene and limonee originate from isoprenoid compounds (terpenoid alcohols). Neophythadiene, typical of tobacco diterpene hydrocarbons originates from thermal decomposition of chlorophyll.
(2) Alcohols and ethers
   Ethanol, soranesol, glycerol, menthol, benzyl alcohol, phenylethyl alcohol, fulfuryl alcohol, 2-methyl furan, 2,5-dimethyl furan.
(3) Organic acids, esters and lactones
   Formic acid, acetic acid, lactic acid, glycolic acid, stearic acid, oleinic acid, linoleic acid. Esters such as acetol acetate and acetol formate. Lactones such as r-butyl lactone.
(4) Aldehydes and ketones
   Formaldehyde, acrolein, furfural, 5-methyl furfural. Ketones such as acetol, diacetyl, sonolane, and cyclo-pentenones.
(5) Phenols
   Phenols such as phenol, cresol, xylenol, catechol, hydroquinone.
(6) Saturated amines
   Alkyl amines, pyrolizine, N-methyl pyrolizine.
(7) Pyridines and pyrazines including alkaloids
   Pyridines in tobacco smoke originate from pyridine alkaloid precursors typified by nicotine. Nitrogen containing compounds such as methylpyrazine, indole, casthol and alpha-pyrolidone.

Chemical compounds characteristic to tobacco such as solanone, norsolana dione and solanofuran originate from terpenes with 14-carbon structure (senbranoids). Tobacco leaf contains carotenoids which is one of the terpenes such as lutine and beta-carotene. Thermal decomposition of carotenoids leads to the formation of beta-ionone, beta-damascone and damacenone, which are called ionone compounds.

Chemical analysis of tobacco smoke shows [Reference 1. S. Ishiguro and S. Sugawara, "Flavor and taste from tobacco smoke", Kouryo (Journal of Flavor), page 31-39, No. 130 (January 1981)] that 46 hydrocarbons, 98 alcohols and esters, 36 aldehydes, 144 ketones and ketols-, 83 esters, 86 lactones, 23 furans, 11 pyrroles, 26 pyridines, 14 pyrazines, 12 amides etc are present.

Leaf tobacco is mainly composed of cellulose, proteins, amino acids, carbohydrates, organic acids and alkaloids. Chemical analysis of leaf tobacco has shown a vast variety of compounds, about 250 compounds counting only the structure. [Reference 2. R.L. Stedman, "Chemical Composition of Tobacco and Tobacco Smoke", Vol. 68, page 153-207 (1969)] describes the following compounds:
(1) Alkanes
   Normal alkanes with 8-35 carbons, iso-alkanes with 27-34 carbons
(2) Alkenes
   Alkenes such as alpha-carotene, beta-carotene and neophytadienes.
(3) Sterols, oxygenated terpenes, other isoprenoids
   Twenty-nine kinds such as campesterol, cholesterol, beta-sistosterol.
(4) Alcohols
   Nineteen kinds such as ethyl alcohol and furfuryl alcohol.
(5) Esters
   Nineteen kinds such as benzyl acetate, di-propyl phthalate, and steryl esters.
(6) Aldehydes, ketones and quinones
   Twenty-seven kinds such as acetaldehyde, isobutyladldehyde, 2-butanone, and 9,10-anthraquinone.
(7) Acids
   Fifty-eight kinds.
(8) Phenols and related compounds
(9) Alkaloids and other bases
   Thirty-seven kinds such as anabasine, costinine, harmane, N-methyl nicotinamide, 2-methyl pyrrolidine, N-methyl pyrrolidine, myosmine, nicotelline, nicotinamide, nicotine, nicotine N-oxide, nicotinic acid, nicotyrine, norharmane, nornicotine, piperidine, pyridine, and pyrrolidine.

Solvent extracts of leaf tobacco have also been analysed in relation to tobacco flavour. According to Reference 3 [Hajime Kaneko, "Tobacco leaf and tobacco flavor", Kouryo (Journal of Flavor), page 23-33, No. 128 (June 1980)] successive extraction of tobacco by dichloromethane, methanol and water shows that the chemical composition of the laminar part of flue-cured tobacco leaf is as follows:

| | |
|---|---|
| Hydrocarbons | 0.66% |
| Solanesol and its esters | 0.99% |
| Non-volatile acid and higher aliphatic acid | 11.3% |
| Polyphenol | 3.19% |
| Glucose, fructose, sucrose | 18.6% |
| Nicotine | 3.4% |
| Proteins | 5.06% |
| Amino acids | 1.0% |
| Cellulose, lignine, pectic substance | 28.1% |
| Ash | 11.1% |
| Oil | 0.72% |

An extract from tobacco leaf contains the following chemical compounds:
(1) Nicotine and related compounds
   Nornicotine which is the major alkaloid of *Nicotiana glutinosa,* anabasine which is the major alkaloid of *Nicotiana glauca,* and 20 other related compounds have been identified in addition to nicotine. In leaf tobacco there are trace amounts of various alkaloids formed from enzymatic and non-enzymatic or oxidative decomposition of nicotine. For example, cotinine is formed through oxidation of nicotine during drying and curing processes.
(2) Saccharides and related compounds
   The main saccharides are sucrose, glucose and fructose. There are several related compounds formed from amino acids and saccharides, for example, 1-deoxy-1-(N-amino acid)-D-Fructose is formed from amino acids and D-glucose upon heating.
(3) Inorganic compounds
   Potassium ion, phosphoric acid ion, ammonium ion, and chlorine ion are present.
(4) Resin compounds on leaf surface
   There are numerous leaf hairs on the surface of tobacco leaf which secrete resin which includes diterpenoids such as duvatriene-1,3-diol and cis-abienol in addition to saturated hydrocarbons. Their derivatives are formed through thermal oxidative thermal and photochemical decomposition during curing, drying and ageing processes. Cembrene, solanone, norsolanadione, and 4-isopropyl-7-oxo-2E-octenoic acid are duvatriene-1,3-diol related compounds and the derivatives formed through decomposition. Cis-abienol is one of the terpenoids with the ravdan structure which undergoes thermal and photochemical decomposition to form a variety of derivatives.
(5) Carotenoids and related compounds
   Eleven carotenoids such as lutine and beta-carotene are known to exist in tobacco leaf. These compounds exist not only in tobacco leaf but also in many plants. 3-Hydroxy-beta-ionol, dehydrololiolide are characteristic to tobacco. Damascenone, 3-hydroxy-beta-damascone, 4-keto-beta-ionone, beta-ionone, 3-hydroxy-beta-iononone, and beta-damascone are also known to be present.
(6) Other extracted constituents
   Hydrocarbon constituents include diterpenoid hydrocarbons such as neophy thadiene, and limonene derived from terpenoid alcohols. Extracts from leaf tobacco contain various higher paraffinic acids and their esters, monoterpenoid alcohol such as geraniol, citronellol and menthol. Lactones such as dihydroactinidiolide, tetrahydroactinidiolide, norambreinolide are also extracted. Nitrogen-containing compounds such as pyrroles, pyrazines, pyridines, amides and imides which derive from chlorophyll and nicotine through their decomposition are also extracted.

Since smoke from cigarettes is considered to be a gas phase extract or the product of thermal decomposition of tobacco, many of the constituents originate from substances found in solvent extracts. A compound which is not thermally decomposed transfers into smoke. Generally speaking, molecular weights of compounds found in gas phase extracts are smaller than those in solvent extracts. Further, gas phase extracts contain derivatives and related compounds such as alkaloids, carotenoids and terpenoids which are found in solvent extracts.

An essential part of the present invention is to provide a medicinal substance for the prevention and treatment of periodontal disease where the substance contains a tobacco extract. As described above, tobacco extract contains numerous chemical compounds. At the present time, it is not specified which compound is effective and it is more plausible to think that there are several such compounds. It is also probable that the medicinal effect on periodontal disease is achieved by the concerted effect of several compounds. Since a number of bacilli cause periodontal disease, it is plausible to think that there are several chemical compounds effective against the disease. The significance of the present invention lies in the discovery that an extract from tobacco is effective for preventing and treating periodontal disease.

In the present invention, the medicinal compounds for preventing and treating periodontal disease are contained in tobacco extracts. Although the medicinally effective compounds are not specified, or it may not be appropriate to do so, there are a few possible candidates, such as alkaloids and their related compounds, the phenolic alcohols, and several other compounds characteristic to tobacco such as diterpenoids and carotenoids. It is mentioned that phenolic alcohols contained in tobacco extracts such as phenol, cresol, xylenol and catechol are known to have a sterilizing effect on some bacilli. The medicinal effect may arise from the concerted effect of these compounds. The present invention has provided a principle for preventing and treating periodontal disease.

It has generally been known that alkaloids (alkaline substances obtained from plants) have a medicinal effect on many kinds of diseases.

It was reported by Dumas in 1937 (refer to "Medical Use of Tobacco- Past and Present-" written by P.S. Larson and H. Silvette, in "Tobacco Alkaloids and Related Compounds": Wenner-Gven Center International Series Vol. 4, pp.3-13, edited by U.S. Von Euler, published by Pergamon Press in 1964), that ointment made from tobacco ash was good for dentifrice. Since the present invention utilizes an extract from tobacco rather than tobacco ash which is the residue after burning, the effective agents are completely different from the report.

Thus, the tobacco extract of the present invention contains alkaloids from tobacco which have the medicinal effects of sterilizing and suppressing the breeding of bacilli which cause periodontal disease. In the present invention the medicinal compounds are not specified, since it is more plausible that several compounds from tobacco extracts exert a concerted or synergistic effect on periodontal disease. If the medicinally effective compounds are to be specified, they are a group of chemical compounds containing nitrogen, found in extracts of tobacco. Chemical compounds characteristic of tobacco, containing nitrogen, are known to be alkaloids. More broadly, the nitrogen-containing compounds include alkaloids and related or derivative compounds. Other nitrogen-containing compounds and their derivatives characteristic to the tobacco extracts include pyridines, nicotine, nornicotine, anabacine, pyrroles, harmanes, indoles, myosmines and other alkaloids, and their related compounds. In the future it may be possible to specify the compound or compounds which are effective against periodontal disease, in which case the compound(s) can be separated and refined from tobacco extracts and used. It is desirable to chemically synthesize the compound or compounds because the medicinal substances are obtained at a higher purity and do not contain undesirable amounts of undesirable chemical substances.

When a tobacco extract with a higher alkaloid concentration is desired, steam extraction is used. An aqueous solution containing tobacco leaf is mixed with an alkaline such as sodium hydroxide or sodium carbonate, and then heated for evaporation. The effluent steam containing alkaloids is collected by condensation and a solution containing alkaloids at a high concentration is obtained.

To obtain a tobacco extract in accordance with the present invention, various kinds of known physico-chemical processes widely used in the chemical and pharmaceutical industries can be applied. If a liquid solvent is used to obtain the extract, water, alcohols such as methyl alcohol and ethyl alcohol, amines such as ethyl amine and aniline, nitriles such as acetonitrile, ethers such as ethyl ether and methyl ethyl ether, organic acids such as acetic acid and oxalic acid, and mixtures thereof can be used. Compounds containing halogens such as dichloro-methane and chloroform can also be used as a solvent. Since water is an excellent solvent in general, it is conveniently used at between 0 and 100 degree centigrade. Hot water is preferably used for faster extraction. Under pressure above one atmosphere, water above 100 degree centigrade can also be used. Water containing inorganic or organic substances can also be used; for example, aqueous solutions of sodium chloride, sodium carbonate, sodium bicarbonate or potassium chloride. Solutions of alcohols can also be used. In order to obtain a tobacco extract using the above mentioned solvents, tobacco is immersed in the solvent and after a certain time the liquid is separated from the residue of tobacco leaf by, for example, filtration, centrifugal separation, or sedimentation. Another method to obtain the tobacco extract is to pass a solvent liquid through a bed packed with tobacco, choosing an appropriate temperature and contact time.

When a tobacco extract with a high alkaloid concentration is desired, steam extraction may be used. An aqueous solution containing tobacco leaf is added to an alkali such as sodium hydroxide, sodium carbonate, potassium hydroxide or potassium carbonate and then heated for evaporation. The effluent steam containing alkaloids is collected by condensation, and a solution containing alkaloids at a high concentrations obtained.

Gas phase extraction is another method for obtaining the tobacco extract of the present invention. Smoking is considered to be gas phase extraction and nicotine and its related compounds are usually found in the smoke. In this case, part of tobacco would burn to raise the temperature of the remainder, resulting in the evaporation of volatile substances and the formation of the products of thermal decomposition. Air, nitrogen, oxygen, helium, argon, hydrogen, steam, and gaseous alcohols can be used as the solvent for gas phase extraction. Supercritical carbon dioxide, which is a state between liquid and gas, can be also used. A gaseous solvent is passed through a bed packed with tobacco at a preset temperature to extract volatile substances from tobacco. The tobacco extract can be collected by, for example, cooling the effluent and condensing volatile matters, filtering, collecting by an electrostatic precipitator or cyclones, or contacting with a solvent such as water. Tobacco extract in the effluent can also be collected through adsorption or absorption, whereby the effluent is contacted with a porous material such as gamma-alumina, silica, silica-alumina, molecular sieves, or active carbons and then the adsorbent is desorbed by heating or is extracted by a solvent. Gas phase extraction using steam is desirable as the collection is done simply by cooling and condensing water.

The raw material for the tobacco extract of the present invention may include (1) tobacco or cigarettes for smoking, (2) leaf tobacco, or the raw material for cigarettes and (3) tobacco plants or a tobacco leaf as the botanical species. The tobacco leaf in (2) may include intermediate products obtained in the process of harvesting, drying, curing, ageing and forming cigarettes. In this specification, the term "tobacco" is used to represent all of these materials.

The present invention is useful in providing a method of applying a tobacco extract to the mouth and teeth. In order to effectively prevent or treat periodontal disease, it is necessary to apply the medicinal substance between the teeth and gums. The substance containing the tobacco extract can be in the form of liquid. A conventional toothbrush can be dipped in the substance and then used for brushing teeth as normal. Since nowadays teeth cleaning agents are supplied as powder, jelly and paste, it is appropriate to make the tobacco extract in these forms. For this, the tobacco extract is mixed with polishing agents, moistening agents, preservatives, gum-like agents and flavourings, and then formed into a paste. Using water as a solvent is one of the most practical methods for obtaining the tobacco extract of the present invention, since the solution can be directly applied to the mouth and teeth. Since an excess amount of water is always necessary in the extracting process, concentration processes such as evaporation by drying and heating may be needed to obtain a solution containing adequate amounts of the medicinal agents. If the evaporation process consumes a lot of energy or time, salts of alkaline earth metals such as calcium hydroxide or barium hydroxide can be added to the solution containing the tobacco extract, which allows faster evaporation of excess water. This is a unique method for obtaining powder containing a tobacco extract. It can then be formed into a paste or jelly.

Rinsing the mouth with a solution containing an appropriate amount of tobacco extract is another method for preventing and treating periodontal disease, when the medicinal agent of the present invention is obtained in the form of liquid. Accordingly, the present invention includes a mouthwash containing a tobacco extract. Another method for applying the medicinal agent to the mouth is to chew a gum containing tobacco extract. A chewing gum (Trade name: Nicoderm) containing nicotine is sold commercially, but the purpose is to help quit smoking, and is entirely different from the objective of the present invention. Since it is not desirable to diffuse the tobacco extract throughout the whole mouth, a needle, pen or thin brush can be used to apply the tobacco extract to a limited area between the teeth and gums. The medicinal material can be applied to the gap between the teeth and gums.

The tobacco extract of the present invention can be used by mixing with commercially available toothpastes. This has the merit of allowing people to follow the normal habit of cleaning the teeth. Examples of the formulations in commercially available toothpaste are as follows:
DENTAL PASTE-C
   Sodium monofluorophosphate, propylene glycol, sodium bicarbonate, water, propylene glycol-1,2, pentasodium triphosphate, tetrasodium pyrophosphate, sodium carbonate, sodium lauryl sulfate, flavour, titanium dioxide, calcium peroxide, sodium saccharin, xanthan gum, and cellulose gum etc.
DENTAL PASTE-P
   Sodium fluoride, water, hydrated silica, trisodium phosphate, sodiumlauryl sulfate, flavouring, sodium phosphate, xanthan gum, sodium saccharin, titanium dioxide, etc.
DENTAL POWDER-S
   Sodium bicarbonate (polishing material), propylene glycol, glycerin (moisturizing agent), sodium lauryl sulfate (foaming agent), peppermint, wintergreen, sodium saccharin, colouring and sodium dehydroacetate (preservative).
DENTAL PASTE-G
   Cetylpyridinium chloride, tocopherol acetate, sodium monofluorophosphate and flavouring.
DENTAL PASTE-M
   Calcium bicarbonate (polishing material), solbit solution (moisturizing agent), sodium chloride, d,1-alpha-tocopherol acetate, sodium lauryl sulfate (foaming agent), sodium carboxymethyl cellulose (viscous agent), flavouring, and sodium saccharin.

The following examples illustrate the present invention in greater detail.

### EXAMPLE 1

About 30 gram of tobacco leaf, obtained from 40 cigarettes (commercial Cigarette M) by removing paper and filter, was placed in a beaker. 1000 ml of water was added and the beaker was left still overnight. The liquid was separated from the tobacco by filtering through a paper towel. About 700 ml of dark brown liquid was obtained (denoted Extract Solution-M1). M1 Extract Solution was heated over an electric heater and concentrated by evaporation to 100 ml.

### EXAMPLE 2

A portion of tobacco leaf was obtained from 40 cigarettes (about 30 gram, commercial Cigarette H) and immersed in 800 ml of water for a day. The liquid was separated from the tobacco leaf by filtration. About 700 ml of a dark brown solution was obtained (H-1). To the H-1 solution 50 gram of sodium chloride was added and Extract Solution H-1S was obtained. The excess sodium chloride was observed to collect at the bottom of the container.

### EXAMPLE 3

About 30 gram of tobacco leaf obtained in a similar way to Example 1 was placed in a coffee server and a total of 1000 ml steam and water was passed through. An Extract solution M-2 with a dark brown colour was obtained and an excess amount of sodium chloride was added (Extract solution M-2X). A very dark brown solution (M-2Y) was obtained by heating 500 ml of the M-2X solution and concentrating to 100 ml.

### EXAMPLE 4

A portion of tobacco leaf from 40 cigarettes (commercial Cigarette M) was immersed in 900 ml of boiling water. After filtration 800 ml of dark brown Extract Solution M-3 was obtained. 400 ml of the M-3 solution was concentrated into 100 ml. 20 gram NaCl was added to obtain M-3Y solution.

### EXAMPLE 5

About 30 ml of Extract Solution M-1X (Example 1) was mixed into 150 gram of a commercial DENTAL PASTE-C and kneaded thoroughly, giving a dental paste containing the tobacco extract (PASTE M-1X-C).

### EXAMPLE 6

Into 160 gram of DENTAL PASTE-P about 90 ml of Extract Solution M-2Y was added and kneaded thoroughly, producing PASTE M-2Y-P.

### EXAMPLE 7

Into 100 gram of DENTAL POWDER-S about 20 ml of the Extract Solution M-3 X was added and kneaded thoroughly. After one day, it was observed that the powder separated from the liquid.

### EXAMPLE 8

A Japanese male of 53 years had pain from a loose tooth caused by periodontal disease which he suffered from for several years. He applied Extract Solution M-1 with a toothbrush to his mouth twice a day (after breakfast and before sleep) for two weeks. After 10 days the pain subsided and the loose tooth became firm. He found that the bad breath also went away in 10 days. Since then he has been using dental PASTE M-1X-C (Example 5) and M-2Y-P (Example 6), and has not suffered from periodontal disease.

### EXAMPLE 9

A Japanese female of 48 years had a light gingivitis for a few years. She had bad breath which she did not notice herself because it was slight. She used Extract Solution H-1 with a toothbrush twice a day for three weeks. Within 10 days people commented that they could no longer detect the bad breath. Furthermore her mouth felt clean and refreshed when she woke up in the morning. It was supposed that the breeding of bacilli in the teeth-gum gap causing the periodontal disease was suppressed during the night by the sterilizing effect of the tobacco extract. Since then she has been using H-1X, M-2Y, M-3Y, M-1X-C and M-2Y-P, and has not suffered from periodontal disease.

### EXAMPLE 10

A Japanese male of 47 years suffering from periodontitis of an early stage had bad breath. He used dental PASTE M-1X-C (Example 5) twice a day for two months. In two weeks his wife commented that the smell had gone. He felt his teeth became firm after a month.

### EXAMPLE 11

A Japanese male of 55 years suffering from periodontitis since his late 40s had bad breath and pain from loose teeth. He started to use dental PASTE M-2Y-P (Example 6), and after 10 days he realized that the odour had disappeared and after a month the pain also went away, leaving his teeth feeling firm.

### EXAMPLE 12

This example describes the preservation of the solutions and the dental pastes containing the tobacco extract. The extract solutions M-1, H-1, M-2 and M-3 were stored in bottles of 150 - 500 ml. It was observed that the surface of the solutions was covered with mould within 7 to 21 days. It was surprising that the mould could grow in these toxic environments and that some bacilli could breed in the concentrated tobacco extracts. When sodium chloride was added to the extract solutions (H-1S, M-2X, M-2Y and M-3Y), the growth of the mould was retarded significantly, and the amount of mould was reduced to one half to one fiftieth compared with those without sodium chloride.

## Claims

1. A tobacco extract for preventing or treating periodontal disease.

2. A tobacco extract according to claim 1, which is an extract of tobacco leaf, part-processed tobacco leaf or tobacco for smoking.

3. One or more chemical compounds characteristic of a tobacco extract for preventing or treating periodontal disease.

4. A tobacco extract according to claim 1 or 2, or a compound or compounds according to claim 3, in which the medicinally active agent is a chemical compound containing nitrogen.

5. A tobacco extract according to claim 1, 2 or 4, or a compound or compounds according to claim 3 or 4, in which the medicinally active agent is one or a mixture of alkaloids, terpenoids and carotenoids.

6. A tobacco extract according to claim 1, 2, 4 or 5, or a compound or compounds according to claims 3, 4 or 5, in which the medicinally active agent is one or a mixture of alkaloids, related compounds and their derivatives.

7. A tobacco extract according to claim 1, 2, 4, 5 or 6, or a compound or compounds according to any of claims 3 to 6, in which the medicinally active agent is one or a mixture of pyridine, nicotine, nornicotine, anabacine, pyrroles, harmanes, indoles, myosmines, related compounds and derivatives.

8. A tobacco extract according to any of claims 1, 2 or 4 to 7, or a compound or compounds according to any of claims 3 to 7, wherein the tobacco extract is contained in materials in the form of solution, paste, jelly, gum or solid.

9. A tobacco extract according to any of claims 1, 2 or 4 to 8, which is extracted by using water or an aqueous solution as a solvent or is obtained in the form of an aqueous solution.

10. A method of producing a tobacco extract according to any of claims 1, 2 or 4 to 9, the method comprising preparing the extract by gas or liquid phase extraction.

11. One or more chemical compounds according to any of claims 3 to 8 which are synthesised by a chemical process.

12. One or more chemical compounds according to any of claims 3 to 8 or 11, in which the chemical compounds contain nitrogen.

13. The use of a tobacco extract in the preparation of a medicament for the prevention or treatment of periodontal disease.

14. The use of one or more chemical compounds characteristic of a tobacco extract in the preparation of a medicament for the prevention or treatment of periodontal disease.

15. The use as claimed in claim 13 or 14, wherein the medicament is in the form of a solution, solid, powder, jelly or paste.

16. The use as claimed in claims 13, 14 or 15, wherein the medicament is applied to parts affected by periodontal disease by brushing, coating, contacting or chewing.
